# EUROPEAN PATENT SPECIFICATION

(11) **EP 1 868 568 B1**
(45) Date of publication and mention of the grant of the patent: **01.10.2008**
(21) Application number: 06761906.4
(22) Date of filing: 28.04.2006
(51) Int. Cl.: A61K 36/16, A61P 9/00, A61P 25/28

(54) **METHOD FOR PREPARING A GINKGO EXTRACT HAVING A REDUCED CONTENT OF 4'-O-METHYL PYRIDOXINE AND/OR BIFLAVONES**
VERFAHREN ZUR HERSTELLUNG EINES GINKGOEXTRAKTS MIT REDUZIERTEM GEHALT AN 4'-O-METHYLPYRIDOXIN UND/ODER BIFLAVONEN
PROCEDE DESTINE A PREPARER UN EXTRAIT DE GINKGO PRESENTANT UNE TENEUR REDUITE EN 4'-O-METHYL-PYRIDOXINE ET/OU EN BIFLAVONES

(30) Priority: 03.05.2005 DE 102005020642
(43) Date of publication of application: 26.12.2007
(73) Proprietor: Dr. Willmar Schwabe GmbH & Co. KG, 76227 Karlsruhe (DE)
(72) Inventor: ERDELMEIER, Clemens, 76139 Karlsruhe (DE); HAUER, Hermann, 76228 Karlsruhe (DE); KOCH, Egon, 76229 Karlsruhe (DE); LANG, Friedrich, 76767 Hagenbach (DE); STUMPF, Karl-Heinz, 76228 Karlsruhe (DE)
(74) Representative: Adam, Holger
(86) International application number: PCT/EP2006/004039
(87) International publication number: WO 2006/117171

(56) References cited:
- EP-A- 0 360 556
- WO-A-99/32129
- DE-A1- 3 940 091
- DATABASE WPI Section Ch, Week 200525 Derwent Publications Ltd., London, GB; Class B04, AN 2005-234134 XP002392815 & CN 1 557 455 A (ZHANG Z) 29 December 2004 (2004-12-29)
- ARENZ ANSGAR ET AL: "Occurrence of neurotoxic 4'-O-methylpyridoxine in Ginkgo biloba leaves, Ginkgo medications and Japanese Ginkgo food" 1996, PLANTA MEDICA, VOL. 62, NR. 6, PAGE(S) 548-551 , XP009070428 ISSN: 0032-0943 page 549, left-hand column

## Description

The present invention relates to a method for preparing an extract from Ginkgo biloba having a reduced content of 4'-O-methyl pyridoxine and biflavones compared to the original extract, characterized by the following steps of the method:
(a) preparing a Ginkgo extract solution in a suitable solvent and
(b) applying the solution to an adsorber resin and eluting the purified extract from the adsorber resin using a suitable solvent, wherein the biflavones to be removed remain on the adsorber resin, and
(c) applying the solution to an acidic ion exchanger and eluting the purified extract from the ion exchanger using a suitable solvent, wherein the 4'-O-methyl pyridoxine to be removed remains on the ion exchanger,
   wherein steps (b) and (c) can also be carried out in reverse order, and
(d) concentrating and drying the extract solution to the dry extract,
wherein the content of 4'-O-methyl pyridoxine in the dry extract is 20 ppm at the most, and
wherein the content of biflavones in the dry extract is 25% of the original value of the original extract at the most.

The present invention further relates to extracts from Ginkgo biloba having a reduced content of 4'-O-methyl pyridoxine and biflavones compared to the original extract, the extracts from Ginkgo biloba being obtainable by the method according to the present invention, as well as to their use.

Since decades, extracts from the leaves of Ginkgo biloba are used as a medicament. They are currently used for the treatment of different types of dementia and symptoms thereof as well as cerebral and peripheral blood circulation disorders. Ingredients, the efficacy is associated with, are terpene lactones (ginkgolides A, B, and C und bilobalide) as well as glycosides of flavones (quercetin, kaempferol and isorhamnetin). However, the leaves of Ginkgo biloba also contain components, which do not contribute to the desired efficacy, but which may be responsible for risks and side effects. In addition to nonpolar plant ingredients, such as ginkgolic acids, those components are 4'-O-methyl pyridoxine and biflavones. In a Ginkgo extract, which is efficacious and at the same time as safe as possible and as low in side effects as possible, these compounds should thus not be present to the largest possible extent. 4'-O-methyl pyridoxine may cause symptoms of poisoning such as convulsive seizures and unconsciousness. Thus, this compound is also referred to as ginkgotoxin. The biflavones contained in ginkgo exhibit an immunotoxic potential and may elicit contact allergies. These biflavones contained in ginkgo are predominantly the compounds amentoflavone, bilobetin, ginkgetin, isoginkgetin and sciadopitysin.

### 4'-O-Methyl pyridoxine:

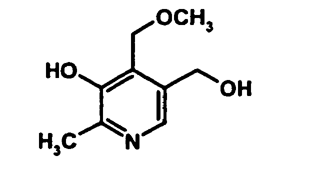

### Biflavones:

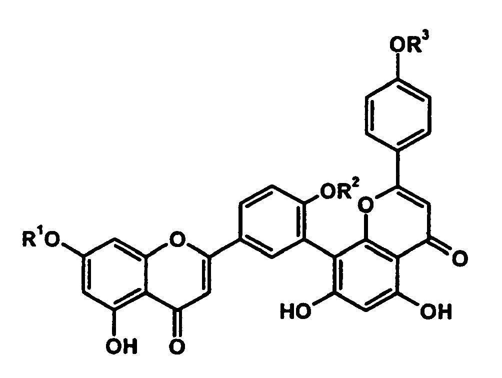

| | |
|---|---|
| Amentoflavone: | R¹ = R² = R³ = H |
| Bilobetin: | R¹ = R³ = H, R² = OCH₃ |
| Ginkgetin: | R³ = H, R¹ = R² = OCH₃ |
| Isoginkgetin: | R¹ = H, R² = R³ = OCH₃ |
| Sciadopitysin: | R¹ = R² = R³ = OCH₃ |

Methods for depleting 4'-O-methyl pyridoxine and biflavones as well as the extracts obtained are already described in EP 1 037 646 B1. Therein, 4'-O-methyl pyridoxine is retained using an acidic cation exchanger and the biflavones are adsorbed on activated carbon. Preferably these depletion steps are carried out after step f) of the method according to EP 1 037 646 B1 (page 3), i.e., after "treating the solution with a lead compound or an insoluble polyamide".

In EP 1 037 646 B1 the depletion of the biflavones and 4'-O-methyl pyridoxine is carried out at a stage of the method (step f)), wherein relatively high contents of lead salts and ammonium salts are present such that an increased amount of ion exchanger has to be employed.

However, this method is limited to a complex sequence of several work-up steps.

Moreover, the depletion is merely described with one single ion exchanger (Merck I) in the case of 4'-O-methyl pyridoxine and with one single adsorbent (active carbon) in the case of biflavones. For this purpose, active carbon has the drawback that it typically does not bind in a very selective manner and cannot be regenerated.

Thus, it is the object underlying the present invention to provide a method for preparing Ginkgo extracts, wherein 4'-O-methyl pyridoxine and biflavones can be depleted to the largest extent possible compared to the original extract and which can additionally be carried out in a simple and cost-efficient manner. A further subject of the present invention are Ginkgo extracts, which are obtainable according to this method.

This object could be solved by separating the biflavones by adsorber resins and separating 4'-O-methyl pyridoxine via acidic ion exchangers.

In the method according to the present invention, the following method steps are performed:
(a) preparing a Ginkgo extract solution in a suitable solvent, as well as
(b) applying the solution to an adsorber resin and eluting the purified extract from the adsorber resin using a suitable solvent, wherein the biflavones to be removed remain on the adsorber resin and may be eluted from the resin using organic solvents, such as acetone, so that the resin can be employed again, and
(c) applying the solution to an acidic ion exchanger and eluting the purified extract from the ion exchanger using a suitable solvent, wherein the 4'-O-methyl pyridoxine to be removed remains on the ion exchanger and may be washed from the ion exchanger using an acid such as aqueous hydrochloric acid, so that the ion exchanger can be employed again,
   wherein steps (b) and (c) can also be carried out in reverse order, and
(d) concentrating and drying the extract solution to the dry extract,
wherein the content of 4'-O-methyl pyridoxine in the dry extract is 20 ppm at the most, and
wherein the content of biflavones in the dry extract is 25% of the original value of the original extract at the most.

Particularly suitable solvents in steps (a), (b) and (c) are independently aqueous ketones having 3 to 6 carbon atoms (such as acetone, 2-butanon) and alkanols having 1 to 3 carbon atoms (methanol; ethanol, n-propanol and isopropanol), preferably aqueous acetone and ethanol, a concentration of 30 to 60% by weight being preferred. Preferred adsorber resins in step (b) are resins on the basis of optionally substituted styrenes/divinylbenzenes such as Diaion HP-20, HP-21 or Sepabeads SP-207 and SP-850. Particularly preferred adsorber resins are copolymers on the basis of styrene and divinylbenzene and copolymers on the basis of brominated styrene and divinylbenzene. Preferred ion exchangers in step (c) are strongly acidic ion exchangers such as Merck I or Amberlite IR-120.

The original extract used to determine the original value is obtained from the original solution (Ginkgo extract solution according to step (a)) by drying to the dry extract.

In step (b) of the method according to the present invention, the amount of the resin used as well as the polarity or the composition of the solvent used have to be adjusted such that the desired components of the extract are eluted from the resin to the largest possible extent first, whereas the biflavones remain on the resin. Thereby, a higher content of water in the solvent leads to a better depletion of the biflavones, i.e., the biflavones remain on the resin. Depending on the solubility of the extract, the water content of the solvent is within the above-mentioned range of 30 to 70 % by weight.

As original extracts (in the form of Ginkgo extract solutions according to step (a) or obtained therefrom) which are to be set free from 4'-O-methyl pyridoxine and biflavones by means of the adsorber resin, the following can be considered:
- single-fold extracts prepared according to methods known per se, for example, according to the European Pharmacopoeia, by extracting dried leaves of Ginkgo biloba using organic solvents or mixtures thereof with water, for example, using ethanol/water mixtures or acetone/water mixtures or
- extracts, the composition of which differs from the underlying single-fold extract due to one or more additional method steps to a greater or lesser extent (socalled special extracts).

The latter extracts can be prepared, for example, by a liquid-liquid distribution according to EP 360556 B1 or by some steps according to EP 431535 B1 such as steps (a) to (c) or by the entire method according to EP 431535 B1 or according to US 6117431.

The Ginkgo extract solutions according to step (a) can either be obtained directly from the preparation process of the extract or by dissolving a dry extract or another extract.

A further subject of the present invention are extracts, in particular dry extracts which are obtainable by the method of the present invention and which are characterized by a reduced content of 4'-O-methyl pyridoxine and biflavones compared to the employed original extracts. The contents of 4'-O-methyl pyridoxine are 20 ppm at the most in the case of extracts according to the present invention, preferably 10 ppm at the most, and in particular 5 ppm at the most. The contents of biflavones are 25% of the original value of the original extract at the most, preferably 11 % at the most, and in particular 6% at the most.

According to the European Pharmacopoeia dry extracts generally have a dry residue of at least 95% by weight.

The extracts according to the present invention can be administered in the form of powders, granules, tablets, dragées (coated tablets) or capsules, preferably orally. In order to prepare tablets, the extract is mixed with suitable pharmaceutically acceptable adjuvants, such as lactose, cellulose, silicon dioxide, croscarmellose and magnesium stearate and pressed into tablets which are optionally provided with a suitable coating made of, for example, hydroxymethylcellulose, polyethyleneglycol, pigments (such as titanium dioxide, iron oxide) and talcum. The extract according to the present invention can also be filled into capsules, optionally under the addition of adjuvants such as stabilizers, fillers and the like. The dosis is such that 1 of 2000 mg, preferably 50-1000 mg, and particularly preferred 100-500 mg of the extract are administered per day.

Moreover, subject of the present invention are medicaments, food products or other preparations which contain these extracts optionally in combination with other substances such as active ingredients and/or adjuvants. The term "food product" as used herein particularly refers to dietetic food products, dietary supplement products as well as medical food and dietary supplements.

### Examples

### Original solution for Comparative Examples 1 to 3 and Example 1 according to the present invention

200 g dried and ground leaves of Ginkgo biloba were extracted twice using each time 7 times their weight (w/w) made up of ethanol/water 60/40 (w/w) at a temperature of about 50°C and filtered.

### Comparative Example 1 (preparation of a single-fold extract as the original extract)

25% of the original solution obtained above were concentrated under reduced pressure and freeze-dried: 14.0 g (28.0% based on the dried leaves).

### Comparative Example 2 (removal of biflavones)

25% of the original solution obtained above were applied to a column with 100 ml Sepabeads SP-850 adsorber resin and eluted with 300 ml 40% by weight ethanol. The eluate was concentrated under reduced pressure and freeze-dried: 11.5 g (23.0% based on the dried leaves).

### Comparative Example 3 (removal of 4'-O-methyl pyridoxine)

25% of the original solution obtained above were applied to a column with 20 ml strongly acidic ion exchanger Amberlite IR-120 and eluted with 60 ml 60% by weight ethanol. The eluate was concentrated under reduced pressure and freeze-dried: 13.0 g (26.0% based on the dried leaves).

### Example 1 according to the invention (removal of biflavones and 4'-O-methyl pyridoxine)

25% of the original solution obtained above were applied to a first column with 100 ml Sepabeads SP-850 adsorber resin and eluted with 300 ml 40% by weight ethanol.

The eluate was applied to a second column with 20 ml strongly acidic ion exchanger Amberlite IR-120 and eluted with 100 ml 40% by weight ethanol. The resulting solution was concentrated under reduced pressure and freeze-dried: 10.1 g (20.2% based on the dried leaves).

### Original solution for Comparative Examples 4 to 6 and Example 2 according to the present invention

200g dried and ground leaves of Ginkgo biloba were extracted twice using each time seven times their weight (w/w) made of ethanol/water 60/40 (w/w) at a temperature of about 50°C and filtered. The extract solution was largely concentrated (145 g), diluted with water to about 400 g and stored for about 19 h at 10°C. The precipitate formed was removed by filtration.

### Comparative Example 4 (preparation of a special extract as the original extract)

25% of the original solution obtained above were concentrated under reduced pressure and freeze-dried: 9.4 g (18.8% based on the dried leaves).

### Comparative Example 5 (removal of biflavones)

25% of the original solution obtained above were applied to a column with 50 ml Sepabeads SP-207 adsorber resin and eluted with 150 ml 40% by weight ethanol. The eluate was concentrated under reduced pressure and freeze-dried: 9.4 g (18.8% based on the dried leaves).

### Comparative Example 6 (removal of 4'-O-methyl pyridoxine)

25% of the original solution obtained above were applied to a column with 20 ml strongly acidic ion exchanger Amberlite IR-120 and eluted with 100 ml 40 % by weight ethanol. The eluate was concentrated under reduced pressure and freeze-dried: 8.7 g (17.4% based on the dried leaves).

### Example 2 according to the present invention (removal of biflavones and 4'-O-methyl pyridoxine)

25% of the original solution obtained above were applied to a first column with 50 ml Sepabeads SP-207 adsorber resin and eluted with 150 ml 40% by weight ethanol. The eluate was applied to a second column with 20 ml strongly acidic ion exchanger Amberlite IR-120 and eluted with 100 ml 40% by weight ethanol. The resulting solution was concentrated under reduced pressure and freeze-dried: 8.3 g (16.6% based on the dried leaves).

The biflavone contents and the 4'-O-methyl pyridoxine contents of the resulting extracts can be taken from the following table. It is to be seen that the sum of the biflavones in Examples 1 and 2 according to the present invention is significantly lower than in the corresponding Comparative Examples 1 and 4, respectively. Analogously, the contents of 4'-O-methyl pyridoxine are significantly reduced in Examples 1 and 2 according to the present invention compared to the corresponding Comparative Examples 1 and 4, respectively.

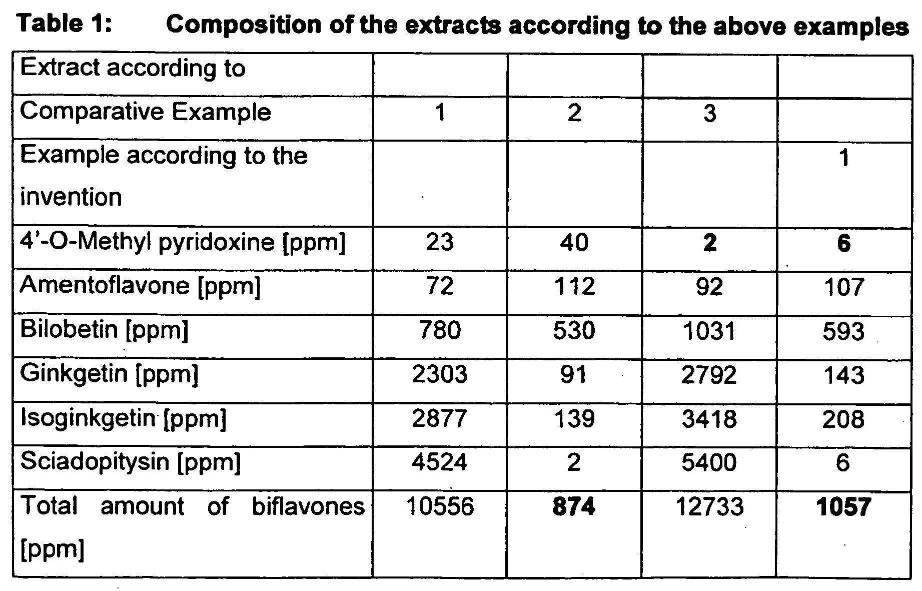

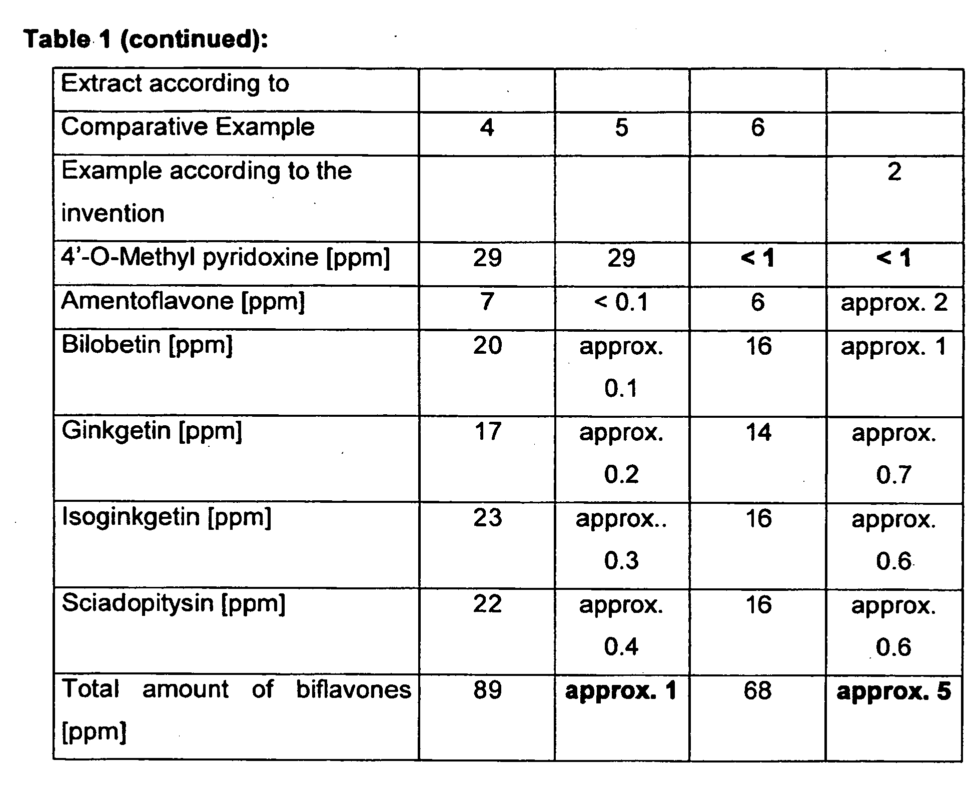

## Claims

1. Method for preparing an extract from Ginkgo biloba having a reduced content of 4'-O-methyl pyridoxine and biflavones compared to the original extract, **characterized by** the following method steps:
(a) preparing a Ginkgo extract solution in a solvent, and
(b) applying the solution to an adsorber resin and eluting the purified extract from the adsorber resin using a solvent, wherein the biflavones to be removed remain on the adsorber resin,
and
(c) applying the solution to an acidic ion exchanger and eluting the purified extract from the ion exchanger using a solvent, wherein the 4'-O-methyl pyridoxine to be removed remains on the ion exchanger,
wherein steps (b) and (c) can also be carried out in reverse order
and
(d) concentrating and drying the extract solution to the dry extract,
wherein the content of 4'-O-methyl pyridoxine in the dry extract is 20 ppm at the most, and
wherein the content of biflavones in the dry extract is 25% of the original value of the original extract at the most.

2. Method according to claim 1, wherein the solvent in steps (a), (b) and (c) is independently selected from an aqueous alkanol having 1 to 3 carbon atoms
and an aqueous ketone having 3 to 6 carbon atoms.

3. Method according to claim 2, wherein the alkanol is methanol, ethanol, n-propanol or isopropanol and the ketone is acetone.

4. Method according to claim 2 or 3, wherein the water content is in a range from 30 to 70% by weight.

5. Method according to any one of claims 2 to 4, wherein the water content of the solvent in steps (a), (b) and (c) is equal or different.

6. Method according to any one of claims 1 to 5, wherein the adsorber resin is a copolymer on the basis of styrene and divinylbenzene.

7. Method according to any one of claims 1 to 5, wherein the adsorber resin is a copolymer on the basis of brominated styrene and divinylbenzene.

8. Method according to any one of claims 1 to 5, wherein the ion exchanger is a strongly acidic ion exchanger.

9. Extract from Ginkgo biloba having a reduced content of 4'-O-methyl pyridoxine and biflavones compared to the original extract, which is obtainable according to the method according to any one of claims 1 to 8, wherein the content of 4'-O-methyl pyridoxine in the dry extract is 20 ppm at the most, and wherein the content of biflavones in the dry extract is 25% of the original value of the original extract at the most.

10. Extract according to claim 9, wherein the content of 4'-O-methyl pyridoxine is 10 ppm at the most.

11. Extract according to claim 9, wherein the content of 4'-O-methyl pyridoxine is 5 ppm at the most.

12. Extract according to any one of claims 9 to 11, wherein the content of biflavones is 11 % of the original value of the original extract at the most.

13. Extract according to any one of claims 9 to 11, wherein the content of biflavones is 6% of the original value of the original extract at the most.

14. Use of the extract according to any one of claims 9 to 13 for the preparation of a medicament, food product or other preparation for the treatment of dementia and symptoms thereof and/or cerebral and peripheral blood circulation disorders.

15. Medicament, food product or other preparation **characterized by** a content of a Gingko extract according to any one of claims 9 to 13.

## Patentansprüche

1. Verfahren zur Herstellung eines Extrakts von Gingko biloba, der einen reduzierten Gehalt an 4'-O-methylpyridoxin und Biflavonen im Vergleich zu dem ursprünglichen Extrakt hat, **gekennzeichnet durch** die folgenden Verfahrensschritte:
(a) Herstellen einer Gingkoextraktlösung in einem Lösungsmittel
und
(b) Auftragen der Lösung auf ein Adsorberharz und Eluieren des gereinigten Extrakts von dem Adsorberharz unter Verwendung eines Lösungsmittels, wobei die zu entfernenden Biflavone an dem Adsorberharz zurückbleiben,
und
(c) Auftragen der Lösung auf einen sauren Ionenaustauscher und Eluieren des gereinigten Extrakts von dem Ionenaustauscher unter Verwendung eines Lösungsmittels, wobei das zu entfernende 4'-O-methylpyridoxin an dem Ionenaustauscher zurückbleibt,
wobei die Schritte (b) und (c) auch in umgekehrter Reihenfolge durchgeführt werden können,
und
(d) Konzentrieren und Trocknen der Extraktlösung zu dem Trockenextrakt,
wobei der Gehalt an 4'-O-methylpyridoxin in dem Trockenextrakt höchstens 20 ppm ist und
wobei der Gehalt an Biflavonen in dem Trockenextrakt höchstens 25% des ursprünglichen Werts des ursprünglichen Extrakts ist.

2. Verfahren nach Anspruch 1, wobei das Lösungsmittel in den Schritten (a), (b) und (c) unabhängig ausgewählt wird aus einem wässrigen Alkanol mit 1 bis 3 Kohlenstoffatomen und einem wässrigen Keton mit 3 bis 6 Kohlenstoffatomen.

3. Verfahren nach Anspruch 2, wobei das Alkanol Methanol, Ethanol, n-Propanol oder Isopropanol ist und das Keton Aceton ist.

4. Verfahren nach Anspruch 2 oder 3, wobei der Wassergehalt in einem Bereich von 30 bis 70 Gew.-% liegt.

5. Verfahren nach einem der Ansprüche 2 bis 4, wobei der Wassergehalt des Lösungsmittels in den Schritten (a), (b) und (c) gleich oder unterschiedlich ist.

6. Verfahren nach einem der Ansprüche 1 bis 5, wobei das Adsorberharz ein Copolymer auf der Basis von Styrol und Divinylbenzol ist.

7. Verfahren nach einem der Ansprüche 1 bis 5, wobei das Adsorberharz ein Copolymer auf der Basis von bromiertem Styrol und Divinylbenzol ist.

8. Verfahren nach einem der Ansprüche 1 bis 5, wobei der Ionenaustauscher ein stark saurer Ionenaustauscher ist.

9. Extrakt von Gingko biloba, der einen reduzierten Gehalt an 4'-O-methylpyridoxin und Biflavonen im Vergleich zu dem ursprünglichen Extrakt hat, der nach dem Verfahren nach einem der Ansprüche 1 bis 8 erhältlich ist, wobei der Gehalt an 4'-O-methylpyridoxin in dem Trockenextrakt höchstens 20 ppm ist und wobei der Gehalt an Biflavonen in dem Trockenextrakt höchstens 25% des ursprünglichen Werts des ursprünglichen Extrakts ist.

10. Extrakt nach Anspruch 9, wobei der Gehalt an 4'-O-methylpyridoxin höchstens 10 ppm ist.

11. Extrakt nach Anspruch 9, wobei der Gehalt an 4'-O-methylpyridoxin höchstens 5 ppm ist.

12. Extrakt nach einem der Ansprüche 9 bis 11, wobei der Gehalt an Biflavonen höchstens 11% des ursprünglichen Werts des ursprünglichen Extrakts ist.

13. Extrakt nach einem der Ansprüche 9 bis 11, wobei der Gehalt an Biflavonen höchstens 6% des ursprünglichen Werts des ursprünglichen Extrakts ist.

14. Verwendung des Extrakts nach einem der Ansprüche 9 bis 13 für die Herstellung eines Medikaments, eines Nahrungsmittelproduktes oder einer anderen Präparation für die Behandlung von Demenz und Symptomen davon und/oder von Störungen des cerebralen und peripheren Blutkreislaufs.

15. Medikament, Nahrungsmittelprodukt oder andere Präparation, **gekennzeichnet durch** einen Gehalt an einem Gingkoextrakt nach einem der Ansprüche 9 bis 13.

## Revendications

1. Un procédé destiné à préparer un extrait de Ginkgo biloba présentant une teneur réduite en 4'-O-méthyle-pyridoxine et biflavones en comparaison à l'extrait original **caractérisé par** les étapes de procédé suivantes .
(a) on prépare une solution d'extrait de Ginkgo dans un solvant et
(b) on applique la solution à une résine adsorbante et on élue l'extrait purifié de la résine adsorbante en utilisant un solvant, les biflavones à éliminer restant à la résine adsorbante et
(c) on applique la solution à une résine échangeuse d'ions acide et on élue l'extrait purifié de la résine échangeuse d'ions en utilisant un solvant, 4'-O-méthyle-pyridoxine à éliminer restant à la résine adsorbante,
dans lequel les étapes (b) et (c) peuvent réaliser aussi en séquence inverse et
(d) on concentre et sèche la solution d'extrait à l'extrait sec,
dans lequel la teneur en 4'-O-méthyle-pyridoxine dans l'extrait sec est au maximum 20 ppm et dans lequel la teneur en biflavones dans l'extrait sec est au maximum 25 % de la valeur originale de l'extrait original.

2. Le procédé selon la revendication 1, dans lequel le solvant dans les étapes (a), (b) et (c) est indépendamment choisi par un alcanol aqueux de 1 à 3 atomes de carbone et une cétone aqueuse 3 à 6 atomes de carbone.

3. Le procédé selon la revendication 2, dans lequel l'alcanol est le méthanol, l'éthanol, le propanol ou l'isopropanol et la cétone est l'acétone.

4. Le procédé selon la revendication 2 ou 3, dans lequel la teneur en eau est comprise dans la gamme de 30 à 70 % en poids.

5. Le procédé selon l'une quelconque des revendications 2 à 4, dans lequel la teneur en eau est identique ou différente dans les étapes (a), (b) et (c).

6. Le procédé selon l'une quelconque des revendications 1 à 5, dans lequel la résine adsorbante est un copolymère à base de styrène et de benzène divinylique.

7. Le procédé selon l'une quelconque des revendications 1 à 5, dans lequel la résine adsorbante est un copolymère à base de styrène bromé et de benzène divinylique.

8. Le procédé selon l'une quelconque des revendications 1 à 5, dans lequel la résine échangeuse d'ions est une résine échangeuse d'ions fortement acide.

9. Un extrait de Ginkgo biloba présentant une teneur réduite en 4'-O-méthyle-pyridoxine et biflavones en comparaison à l'extrait original, qui peut être obtenu par le procédé selon l'une quelconque des revendications 1 à 8, dans lequel la teneur en 4'-O-méthyle-pyridoxine est au maximum 20 ppm dans l'extrait sec et dans lequel la teneur en biflavones dans l'extrait sec est au maximum 25 % de la valeur originale de l'extrait original.

10. L'extrait selon la revendication 9, dans lequel la teneur en 4'-O-méthyle-pyridoxine est au maximum 10 ppm.

11. L'extrait selon la revendication 9, dans lequel la teneur en 4'-O-méthyle-pyridoxine est au maximum 5 ppm.

12. L'extrait selon l'une quelconque des revendications 9 à 11, dans lequel la teneur en biflavones est au maximum 11 % de la valeur originale de l'extrait original.

13. L'extrait selon l'une quelconque des revendications 9 à 11, dans lequel la teneur en biflavones est au maximum 6 % de la valeur originale de l'extrait original.

14. Utilisation de l'extrait selon l'une quelconque des revendications 9 à 13 pour la fabrication d'un médicament, d'un produit alimentaire ou d'une autre préparation destiné(e) au traitement de la démence et des symptômes de la démence et/ou des perturbations de la circulation sanguine tant périphérique que cérébrale.

15. Un médicament, un produit alimentaire ou une autre préparation **caractérisé(e)** en ce qu'il/elle contient une teneur en Ginkgo extrait selon l'une quelconque des revendications 9 à 13.
